(19) Europäisches Patentamt
European Patent Office
Office européen des brevets

(11) Veröffentlichungsnummer: **0 438 574 B1**

(12) **EUROPÄISCHE PATENTSCHRIFT**

(45) Veröffentlichungstag der Patentschrift: **25.01.95**

(51) Int. Cl.[6]: **C09K 19/34**, C07D 239/26, C07D 319/06

(21) Anmeldenummer: **90912422.4**

(22) Anmeldetag: **10.08.90**

(86) Internationale Anmeldenummer:
**PCT/EP90/01318**

(87) Internationale Veröffentlichungsnummer:
**WO 91/02780 (07.03.91 91/06)**

# (54) 2,5-DISUBSTITUIERTE HETEROCYCLEN UND FLÜSSIGKRISTALLINES MEDIUM.

(30) Priorität: **12.08.89 DE 3926745**
**05.09.89 DE 3929421**

(43) Veröffentlichungstag der Anmeldung:
**31.07.91 Patentblatt 91/31**

(45) Bekanntmachung des Hinweises auf die Patenterteilung:
**25.01.95 Patentblatt 95/04**

(84) Benannte Vertragsstaaten:
**DE GB**

(56) Entgegenhaltungen:
**EP-A- 0 315 014**
**DE-A- 3 504 866**
**DE-A- 3 705 071**

(73) Patentinhaber: **MERCK PATENT GmbH**
**Postfach,**
**Frankfurter Strasse 250**
**D-64271 Darmstadt (DE)**

(72) Erfinder: **WÄCHTLER, Andreas**
**Goethestr. 34**
**D-6103 Griesheim (DE)**
Erfinder: **HITTICH, Reinhard**
**Am Kirchberg 11**
**D-6101 Modautal 1 (DE)**
Erfinder: **POETSCH, Eike**
**Am Buchwald 4**
**D-6109 Mühltal (DE)**
Erfinder: **KRAUSE, Joachim**
**Samuel-Morse-Str. 14**
**D-6110 Dieburg (DE)**
Erfinder: **FINKENZELLER, Ulrich**
**Waldpfad 74**
**D-6831 Plankstadt (DE)**
Erfinder: **COATES, David**
**87 Sopwith Crescent**
**Merley**
**Wimborne**
**Dorset BH21 3SW (GB)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 438 574 B1

## Beschreibung

Die Erfindung betrifft neue 2,5-disubstituierte Heterocyclen der Formel I

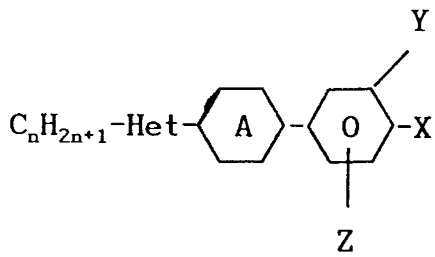

I

worin

n 1 bis 10,

Het

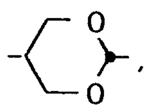, 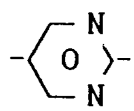 oder 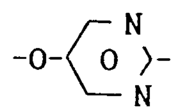,

Ring A trans-1,4-Cyclohexylen oder im Falle

Het = 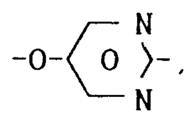,

X = $-OCF_3$ oder $-OCHF_2$ und/oder Y = Z = F auch 1,4-Phenylen,

X F, Cl, $-CF_3$, $-OCF_3$ oder $-OCHF_2$ und

Y und Z jeweils unabhängig voneinander H oder F

bedeuten, wobei im Falle X = F Y = F bedeutet.

Aus der DE-OS 32 07 114 sind ähnliche Verbindungen der Formel

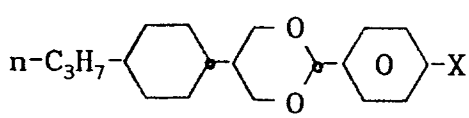

bekannt, worin X F, Cl, Br, $CF_3$ oder CN bedeutet. Diese Verbindungen werden jedoch nicht extremen Anforderungen an die chemische Stabilität gerecht, wie sie beispielsweise für Anzeigen mit aktiver Matrix gefordert werden.

Aus der EP-OS 0 193 191 sind ähnliche Verbindungen der Formel

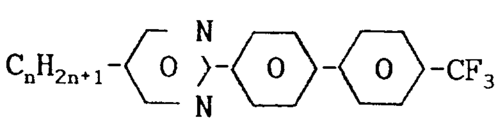

bekannt, worin n 2 bis 6 bedeutet. Diese Verbindungen zeigen jedoch Schmelzpunkte von deutlich mehr als 125 °C und ausschließlich smektische Phasen.

Aus der DE-A-35 04 866 sind ähnliche Verbindungen der Formel

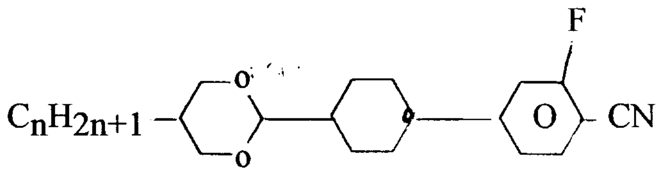

bekannt. Eine allgemeine Formel für weitere ähnliche Verbindungen mit terminaler Substitution durch Fluor, jedoch ohne zusätzliche laterale Substitution durch Fluor ist in EP-A-0 315 014 offenbart.

Die Verbindungen der Formel I können wie ähnliche, z.B. aus den DE-OS 29 44 905 und 27 02 591 bekannte Verbindungen als Komponenten flüssigkristalliner Medien verwendet werden, insbesondere für Anzeigen, die auf dem Prinzip der verdrillten Zelle beruhen.

Die bisher für diesen Zweck eingesetzten Substanzen besitzen sämtliche gewisse Nachteile, beispielsweise zu hohe Schmelzpunkte, zu niedrige Klärpunkte, zu geringe Stabilität gegenüber der Einwirkung von Wärme, Licht oder elektrischen Feldern, zu niedriger elektrischer Widerstand, zu hohe Temperaturabhängigkeit der Schwellenspannung.

Insbesondere bei Anzeigen vom Supertwisttyp (STN) mit Verdrillungswinkeln von deutlich mehr als 220 °C oder bei Anzeigen mit aktiver Matrix weisen die bisher eingesetzten Materialien Nachteile auf.

Der Erfindung lag die Aufgabe zugrunde, neue flüssigkristalline Verbindungen aufzufinden, die als Komponenten flüssigkristalliner Medien geeignet sind, insbesondere für nematische Medien mit positiver dielektrischer Anisotropie, und die die Nachteile der bekannten Verbindungen nicht oder nur in geringerem Maße zeigen. Diese Aufgabe wurde durch die Bereitstellung der neuen Verbindungen der Formel I gelöst.

Es wurde gefunden, daß die Verbindungen der Formel I vorzüglich als Komponenten flüssigkristalliner Medien geeignet sind. Insbesondere sind mit ihrer Hilfe flüssigkristalline Medien mit weiten nematischen Bereichen, hervorragender Nematogenität bis zu tiefen Temperaturen, hervorragender chemischer Stabilität, ausgeprägtem $\epsilon_\perp$ bei positiver dielektrischer Anisotropie, geringer Temperaturabhängigkeit der Schwellenspannung und/oder kleiner optischer Anisotropie erhältlich. Die neuen Verbindungen zeigen außerdem eine gute Löslichkeit für andere Komponenten derartiger Medien und hohe positive dielektrische Anisotropie bei gleichzeitig günstiger Viskosität.

Die Verbindungen der Formel I ermöglichen sowohl STN-Anzeigen mit sehr hoher Steilheit der elektrooptischen Kennlinie als auch Anzeigen mit aktiver Matrix mit hervorragender Langzeitstabilität.

Die Verbindungen der Formel I sind in reinem Zustand farblos und bilden flüssigkristalline Mesophasen in einem für die elektrooptische Verwendung günstig gelegenen Temperaturbereich.

Gegenstand der Erfindung sind somit die Verbindungen der Formel I sowie die Verwendung der Verbindungen der Formel I als Komponenten flüssigkristalliner Medien, flüssigkristalline Medien mit einem Gehalt an mindestens einer Verbindung der Formel I und elektrooptische Anzeigen, die derartige Medien enthalten.

Vor- und nachstehend haben n, A, X, Y und Z die angegebene Bedeutung, sofern nicht ausdrücklich etwas anderes vermerkt ist.

In den Verbindungen der Formel I sind die Alkylgruppen $C_nH_{2n+1}$ vorzugsweise geradkettig. Dementsprechend bedeutet $C_nH_{2n+1}$ vorzugsweise Methyl, Ethyl, n-Propyl, n-Butyl, n-Pentyl, n-Hexyl, n-Heptyl, n-Octyl, n-Nonyl oder n-Decyl. n ist vorzugsweise 2, 3, 4 oder 5.

Verbindungen der Formel I mit verzweigten Alkylgruppen können gelegentlich wegen einer besseren Löslichkeit in den üblichen flüssigkristallinen Basismaterialien von Bedeutung sein, insbesondere aber als chirale Dotierstoffe, wenn sie optisch aktiv sind. Verzweigte Gruppen dieser Art enthalten in der Regel nicht mehr als eine Kettenverzweigung. Bevorzugte verzweigte Alkylreste sind Isopropyl, 2-Butyl (= 1-Methylpropyl), Isobutyl (= 2-Methyl-propyl), 2-Methylbutyl, Isopentyl (= 3-Methylbutyl), 2-Methylpentyl, 3-Methylpentyl, 2-Heptyl (= 1-Methylhexyl), 2-Octyl (= 1-Methylheptyl), 2-Ethylhexyl.

Der Rest

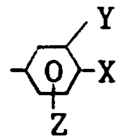

ist vorzugsweise

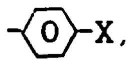, 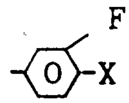 oder 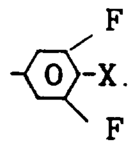.

X ist vorzugsweise F, Cl, $-CF_3$, $-OCHF_2$ oder $-OCF_3$.

Besonders bevorzugt sind die 2-(trans-4-Phenylcyclohexyl)-5-alkyl-1,3-dioxane der Formel Ia

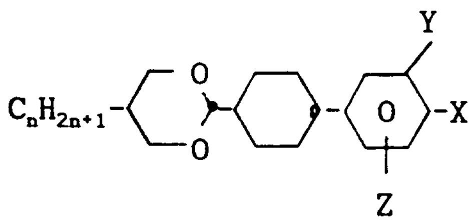 Ia

worin n 1 bis 10, X F, Cl, $-CF_3$, $-OCF_3$ oder $-OCHF_2$ und Y und Z jeweils unabhängig voneinander H oder F bedeuten, sowie die 2,5-disubstituierten Pyrimidine der Formel Ib

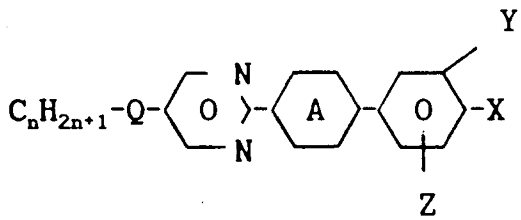 Ib

worin n 1 bis 10, Q -O- oder eine Einfachbindung, A trans-1,4-Cyclohexylen oder 1,4-Phenylen, X F, Cl, $-CF_3$, $-OCF_3$ oder $-OCHF_2$ und Y und Z jeweils unabhängig voneinander H oder F bedeuten.

Q ist vorzugsweise eine Einfachbindung.

Sowohl in Ia als auch in Ib stellt im Falle von X = F die Gruppe Y ein Fluoratom dar.

Die Verbindungen der Formel I werden im übrigen nach an sich bekannten Methoden hergestellt, wie sie in der Literatur (z.B. in den Standardwerken wie Houben-Weyl, Methoden der Organischen Chemie, Georg-Thieme-Verlag, Stuttgart) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsstoffe können gewünschtenfalls auch in situ gebildet werden, derart, daß man sich aus dem Reaktionsgemisch nicht isoliert, sondern sofort weiter zu den Verbindungen der Formel I umsetzt.

Vorzugsweise wird zur Herstellung der 1,3-Dioxane ein Aldehyd der Formel II

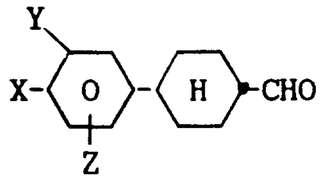

II

worin X, Y und Z die angegebene Bedeutung haben, oder eines seiner funktionellen Derivate mit einem Diol der Formel III

$(HOCH_2)_2\text{-}CH\text{-}C_nH_{2n+1}$ III

worin n die angegebene Bedeutung hat, umgesetzt.

Die Verbindungen der Formeln II und III werden zweckmäßig in Gegenwart eines inerten Lösungsmitteln wie Benzol oder Toluol und/oder eines Katalysators, z.B. einer starken Säure wie Schwefelsäure, Benzol- oder p-Toluolsulfonsäure, bei Temperaturen zwischen etwa 20 und etwa 150, vorzugsweise zwischen 80 und 120°, miteinander umgesetzt. Als reaktionsfähige Derivate der Verbindungen der Formeln II und III eignen sich in erster Linie einfache Acetale.

Die Ausgangsstoffe der Formeln II und III sowie ihre reaktionsfähigen Derivate sind zum Teil bekannt, zum Teil können sie ohne Schwierigkeiten nach Standardverfahren der organischen Chemie aus literaturbekannten Verbindungen hergestellt werden. Beispielsweise sind die Aldehyde der Formel II durch Oxydation entsprechender Alkohole oder durch Reduktion entsprechender Carbonsäuren (oder ihrer Derivate) erhältlich, die Diole der Formel III durch Reduktion entsprechender Diester der Formel $(AlkylOOCCH_2)_2\text{-}CH\text{-}C_nH_{2n+1}$.

Die zur Synthese der Aldehyde der Formel II geeigneten Vorstufen sind beispielsweise nach folgendem Syntheseschema erhältlich:

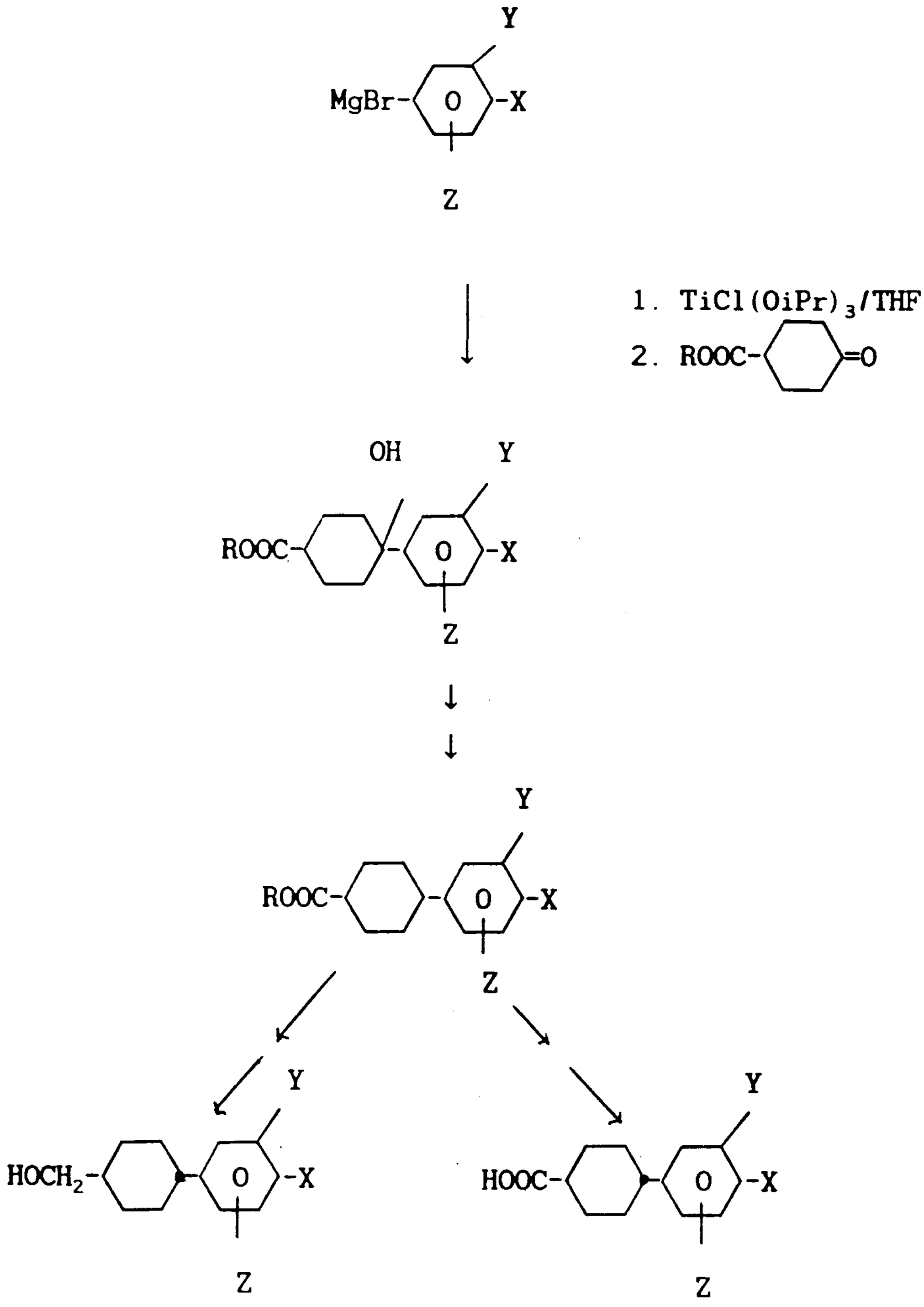


Die aus dem entsprechenden Brombenzol-Derivat erhaltene Grignard-Verbindung wird mit Chlortrialkylorthotitanat bzw. -zirkonat nach WO 87/05599 zu dem tertiären Cyclohexanol umgesetzt. Nach Abspaltung von Wasser, Hydrierung der Doppelbindung und Isomerisierung erhält man nach üblichen Methoden den trans-Cyclohezancarbonsäureester. Aus letzterem erhält man nach üblichen standardverfahren die geeigneten Vorprodukte für die Aldehyde der Formel II.

Zur Herstellung der Pyrimidine werden vorzugsweise entsprechende Amidine gemäß Schema 2 zu den erfindungsgemäßen Verbindungen kondensiert. Die Amidine können wie in Schema 1 gezeigt hergestellt werden.

Schema 1

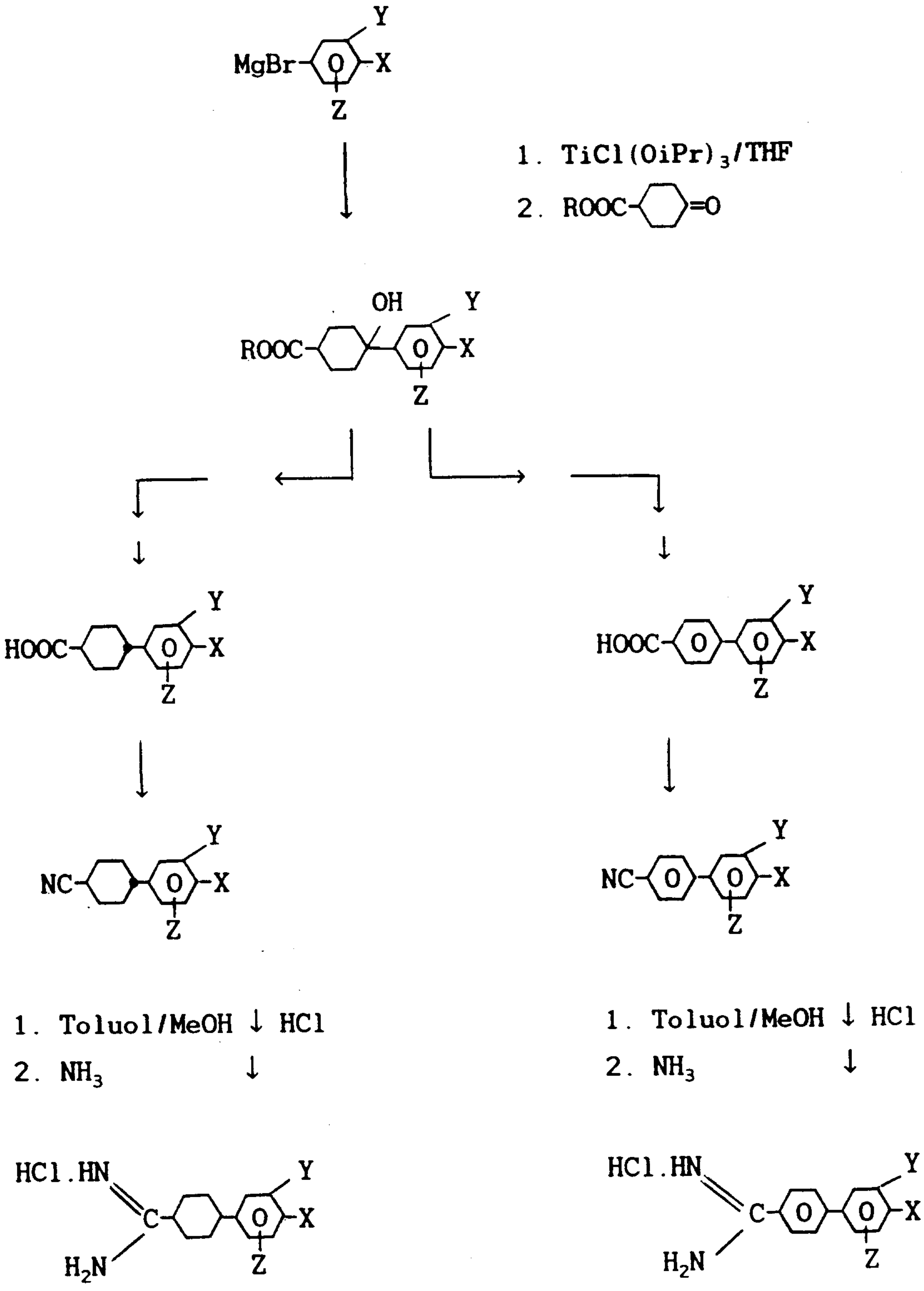
1. TiCl(OiPr)3/THF
2.
1. Toluol/MeOH HCl
2. NH3
1. Toluol/MeOH HCl
2. NH3

Schema 2

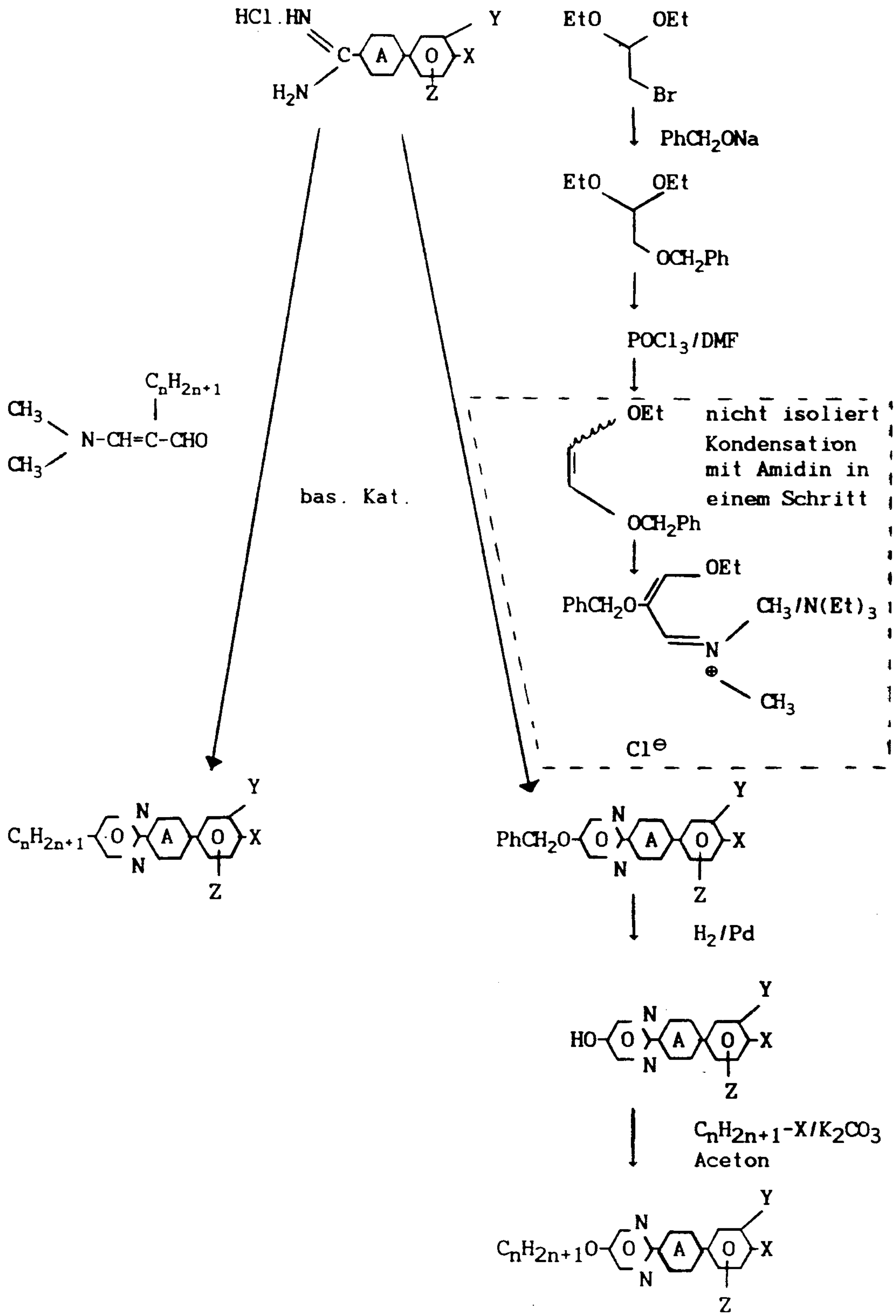


Die aus dem entsprechenden Brombenzol-Derivat erhaltene Grignard-Verbindung wird mit Chlortrialkylorthotitanat bzw. -zirkonat nach WO 87/05599 zu dem tertiären Cyclohexanol umgesetzt. Nach Abspaltung von Wasser, Hydrierung der Doppelbindung und Isomerisierung bzw. Dehydrierung erhält man nach üblichen Methoden den trans-Cyclohexancarbonsäure bzw. die Benzoesäure. Aus letzteren erhält man nach üblichen Standardverfahren die geeigneten Vorstufen für die Amidine.

Die erfindungsgemäßen flüssigkristallinen Medien enthalten vorzugsweise neben einer oder mehreren erfindungsgemäßen Verbindungen als weitere Bestandteile 2 bis 40, insbesondere 4 bis 30 Komponenten. Ganz besonders bevorzugt enthalten diese Medien neben einer oder mehreren erfindungsgemäßen Verbindungen 7 bis 25 Komponenten. Diese weiteren Bestandteile werden vorzugsweise ausgewählt aus nematischen oder nematogenen (monotropen oder isotropen) Substanzen, insbesondere Substanzen aus den Klassen der Biphenyle, Terphenyle, Phenyl- oder Cyclohexylbenzoate, Cyclohexancarbonsäurephenyl- oder cyclohexyl-ester, Phenyl- oder Cyclohexylester der Cyclohexylbenzoesäure, Phenyl- oder Cyclohexylester der Cyclohexylcyclohexancarbonsäure, Cyclohexylphenylester der Benzoesäure, der Cyclohexancarbonsäure, bzw. der Cyclohexylcyclohexancarbonsäure, Phenylcyclohexane, Cyclohexylbiphenyle, Phenylcyclohexylcyclohexane, Cyclohexylcyclohexane, Cyclohexylcyclohexene, Cyclohexylcyclohexylcyclohexene, 1,4-Biscyclohexylbenzole, 4,4'-Bis-cyclohexylbiphenyle, Phenyl- oder Cyclohexylpyrimidine, Phenyl- oder Cyclohexylpyridine, Phenyl- oder Cyclohexyldioxane, Phenyl- oder Cyclohexyl-1,3-dithiane, 1,2-Diphenylethane, 1,2-Dicyclohexylethane, 1-Phenyl-2-cyclohexylethane, 1-Cyclohexyl-2-(4-phenyl-cyclohexyl)-ethane, 1-Cyclohexyl-2-biphenylylethane, 1-Phenyl-2-cyclohexylphenylethane und Tolane.

Die 1,4-Phenylengruppen in diesen Verbindungen können auch fluoriert sein.

Die wichtigsten als weitere Bestandteile erfindungsgemäßer Medien in Frage kommenden Verbindungen lassen sich durch die Formeln 1, 2, 3, 4 und 5 charakterisieren:

R'-L-E-R''   1

R'-L-COO-E-R''   2

R'-L-OOC-E-R''   3

R'-L-$CH_2CH_2$-E-R''   4

R'-L-C≡C-E-R''   5

In den Formeln 1, 2, 3, 4 und 5 bedeuten L und E, die gleich oder verschieden sein können, jeweils unabhängig voneinander einen bivalenten Rest aus der aus -Phe-, -Cyc-, -Phe-Phe-, -Phe-Cyc-, -Cyc-Cyc-, -Pyr-, -Dio-, -G-Phe- und -G-Cyc-sowie deren Spiegelbilder gebildeten Gruppe, wobei Phe unsubstituiertes oder durch Fluor substituiertes 1,4-Phenylen, Cyc trans-1,4-Cyclohexylen oder 1,4-Cyclohexenylen, Pyr Pyrimidin-2,5-diyl oder Pyridin-2,5-diyl, Dio 1,3-Dioxan-2,5-diyl und G 2-(trans-1,4-Cyclohexyl)-ethyl, Pyrimidin-2,5-diyl, Pyridin-2,5-diyl oder 1,3-Dioxan-2,5-diyl bedeuten.

Vorzugsweise ist einer der Reste L und E Cyc, Phe oder Pyr. E ist vorzugsweise Cyc, Phe oder Phe-Cyc. Vorzugsweise enthalten die erfindungsgemäßen Medien eine oder mehrere Komponenten ausgewählt aus den Verbindungen der Formeln 1, 2, 3, 4 und 5, worin L und E ausgewählt sind aus der Gruppe Cyc, Phe und Pyr und gleichzeitig eine oder mehrere Komponenten ausgewählt aus den Verbindungen der Formeln 1, 2, 3, 4 und 5, worin einer der Reste L und E ausgewählt ist aus der Gruppe Cyc, Phe und Pyr und der andere Rest ausgewählt ist aus der Gruppe -Phe-Phe-, -Phe-Cyc-, -Cyc-Cyc-, -G-Phe- und -G-Cyc-, und gegebenenfalls eine oder mehrere Komponenten ausgewählt aus den Verbindungen der Formeln 1, 2, 3, 4 und 5, worin die Reste L und E ausgewählt sind aus der Gruppe -Phe-Cyc-, -Cyc-Cyc-, -G-Phe- und -G-Cyc-.

R' und R'' bedeuten in den Verbindungen der Teilformeln 1a, 2a, 3a, 4a und 5a jeweils unabhängig voneinander Alkyl, Alkenyl, Alkoxy, Alkenyloxy oder Alkanoyloxy mit bis zu 8 Kohlenstoffatomen. Bei den meisten dieser Verbindungen sind R' und R'' voneinander verschieden, wobei einer dieser Reste meist Alkyl oder Alkenyl ist. In den Verbindungen der Teilformeln 1b, 2b, 3b, 4b und 5b bedeutet R'' -CN, $-CF_3$, $-OCF_3$, F, Cl oder -NCS; R hat dabei die bei den Verbindungen der Teilformeln 1a bis 5a angegebene Bedeutung und ist vorzugsweise Alkyl oder Alkenyl. Besonders bevorzugt ist R'' ausgewählt aus der Gruppe bestehend aus -F, Cl, $CF_3$ und $-OCF_3$. Aber auch andere Varianten der vorgesehenen Substituenten in den Verbindungen der Formeln 1, 2, 3, 4 und 5 sind gebräuchlich.

Viele solcher Substanzen oder auch Gemische davon sind im Handel erhältlich. Alle diese Substanzen sind nach literaturbekannten Methoden oder in Analogie dazu erhältlich.

Die erfindungsgemäßen Medien enthalten vorzugsweise neben Komponenten aus der Gruppe der Verbindungen 1a, 2a, 3a, 4a und 5a (Gruppe 1) auch Komponenten aus der Gruppe der Verbindungen 1b, 2b, 3b, 4b und 5b (Gruppe 2), deren Anteile vorzugsweise wie folgt sind:

Gruppe 1: 20 bis 90 %, insbesondere 30 bis 90 %,

Gruppe 2: 10 bis 80 %, insbesondere 10 bis 50 %,

wobei die Summe der Anteile der erfindungsgemäßen Verbindungen und der Verbindungen aus den Gruppen 1 und 2 bis zu 100 % ergeben.

Die erfindungsgemäßen Medien enthalten vorzugsweise 1 bis 40 %, insbesondere vorzugsweise 5 bis 30 % an erfindungsgemäßen Verbindungen. Weiterhin bevorzugt sind Medien, enthaltend mehr als 40 %, insbesondere 45 bis 90 % an erfindungegemäßen Verbindungen. Die Medien enthalten vorzugsweise drei, vier oder fünf erfindungsgemäße Verbindungen.

Die Herstellung der erfindungsgemäßen Medien erfolgt in an sich üblicher Weise. In der Regel werden die Komponenten ineinander gelöst, zweckmäßig bei erhöhter Temperatur. Durch geeignete Zusätze können die flüssigkristallinen Phasen nach der Erfindung so modifiziert werden, daß sie in allen bisher bekannt gewordenen Arten von Flüssigkristallanzeigeelementen verwendet werden können.

Derartige Zusätze sind dem Fachmann bekannt und in der Literatur ausführlich beschrieben (H. Kelker/R. Hatz, Handbook of Liquid Crystals, Verlag Chemie, Weinheim, 1980). Beispielsweise können pleochroitische Farbstoffe zur Herstellung farbiger Guest-Host-Systeme oder Substanzen zur Veränderung der dielektrischen Anisotropie, der Viskosität und/oder der Orientierung der nematischen Phasen zugesetzt werden.

Die folgenden Beispiele sollen die Erfindung erläutern, ohne sie zu begrenzen. mp. = Schmelzpunkt, cp. = Klärpunkt. Vor- und nachstehend bedeuten Prozentangaben Gewichtsprozent; alle Temperaturen sind in Grad Celsius angegeben. "Übliche Aufarbeitung" bedeutet: man gibt Wasser hinzu, extrahiert mit Methylenchlorid, trennt ab, trocknet die organische Phase, dampft ein und reinigt das Produkt durch Kristallisation und/oder Chromatographie.

Es bedeuten ferner:

K: Kristallin-fester Zustand, S: smektische Phase (der Index kennzeichnet den Phasentyp), N: nematischer Zustand,

Ch: cholesterische Phase, I: isotrope Phase. Die zwischen zwei Symbolen stehende Zahl gibt die Umwandlungstemperatur in Grad Celsius an.

| | |
|---|---|
| DAST | Diethylaminoschwefeltrifluorid |
| DCC | Dicyclohexylcarhodiimid |
| DDQ | Dichlordicyanobenzochinon |
| DIBALH | Diisobutylaluminiumhydrid |
| DMSO | Dimethylsulfoxid |
| KOT | Kalium-tertiär-butanolat |
| THF | Tetrahydrofuran |
| pTSOH | p-Toluolsulfonsäure |

Beispiel 1

Ein Gemisch von 2,2 g trans-4-(3,4-Difluorphenyl)-cyclohexancarbaldehyd (erhältlich durch Überführung der entsprechenden Säure in das Chlorid und Rosenmund-Reduktion) 1,32 g 2-Butylpropan-1,3-diol, 0,01 g p-Toluolsulfonsäure und 15 ml Toluol wird am Wasserabscheider 3 Std. gekocht, abgekühlt, mit Wasser gewaschen und eingedampft. Man erhält 2-[trans-4-(3,4-Difluorphenyl)-cyclohexyl]-5-butyl-1,3-dioxan.

Beispiele 2 bis 25

Analog Beispiel 1 erhält man aus den entsprechenden Aldehyden und den entsprechenden 1,3-Diolen die folgenden Verbindungen:

| | n | X | Y | Z |
|---|---|---|---|---|
| (2) | 2 | F | F | H |
| (3) | 3 | F | F | H |
| (4) | 5 | F | F | H |
| (5) | 7 | F | F | H |
| (6) | 2 | Cl | H | H |
| (7) | 3 | Cl | H | H |
| (8) | 4 | Cl | H | H |
| (9) | 5 | Cl | H | H |
| (10) | 2 | Cl | F | H |
| (11) | 3 | Cl | F | H |
| (12) | 4 | Cl | F | H |
| (13) | 5 | Cl | F | H |
| (14) | 2 | $CF_3$ | H | H |
| (15) | 3 | $CF_3$ | H | H |
| (16) | 4 | $CF_3$ | H | H |
| (17) | 5 | $CF_3$ | H | H |
| (18) | 2 | $OCF_3$ | H | H |
| (19) | 3 | $OCF_3$ | H | H |
| (20) | 4 | $OCF_3$ | H | H |
| (21) | 5 | $OCF_3$ | H | H |
| (22) | 2 | $OCHF_2$ | H | H |
| (23) | 3 | $OCHF_2$ | H | H |
| (24) | 4 | $OCHF_2$ | H | H |
| (25) | 5 | $OCHF_2$ | H | H |

Beispiel 26

In einer Natriummethylat-Lösung in Methanol gibt man 0,01 mol 4-(3,4-Difluorphenyl)-benzamidinhydrochlorid und 0,01 mol α-Propyl-β-dimethylaminoacrolein und kocht 5 Stunden am Rückfluß. Nach Abdampfen des Methanols nimmt man in Toluol auf und arbeitet wie üblich wäßrig auf. Man erhält 5-n-Propyl-2-[4-(3,4-difluorphenyl)-phenyl]-pyrimidin.

Beispiele 27 bis 74:

Analog Beispiel 26 erhält man aus den entsprechenden Amidinen die folgenden Verbindungen:

| | n | X | Y | Z | Q | A |
|---|---|---|---|---|---|---|
| (27) | 2 | F | F | H | - | -Phe- |
| (28) | 4 | F | F | H | - | -Phe- |
| (29) | 5 | F | F | H | - | -Phe- |
| (30) | 7 | F | F | H | - | -Phe- |
| (31) | 2 | Cl | H | H | - | -Phe- |
| (32) | 3 | Cl | H | H | - | -Phe- |
| (33) | 4 | Cl | H | H | - | -Phe- |
| (34) | 5 | Cl | H | H | - | -Phe- |

| | n | X | Y | Z | Q | A |
|---|---|---|---|---|---|---|
| (35) | 2 | Cl | F | H | - | -Phe- |
| (36) | 3 | Cl | F | H | - | -Phe- |
| (37) | 4 | Cl | F | H | - | -Phe- |
| (38) | 5 | Cl | F | H | - | -Phe- |
| (39) | 2 | $CF_3$ | H | H | - | -Phe- |
| (40) | 3 | $CF_3$ | H | H | - | -Phe- |
| (41) | 4 | $CF_3$ | H | H | - | -Phe- |
| (42) | 5 | $CF_3$ | H | H | - | -Phe- |
| (43) | 2 | $OCF_3$ | H | H | - | -Phe- |
| (44) | 3 | $OCF_3$ | H | H | - | -Phe- |
| (45) | 4 | $OCF_3$ | H | H | - | -Phe- |
| (46) | 5 | $OCF_3$ | H | H | - | -Phe- |
| (47) | 2 | $OCHF_2$ | H | H | - | -Phe- |
| (48) | 3 | $OCHF_2$ | H | H | - | -Phe- |
| (49) | 4 | $OCHF_2$ | H | H | - | -Phe- |
| (50) | 5 | $OCHF_2$ | H | H | - | -Phe- |

| | n | X | Y | Z | Q | A |
|---|---|---|---|---|---|---|
| (51) | 2 | F | F | H | - | -Cyc- |
| (52) | 4 | F | F | H | - | -Cyc- |
| (53) | 5 | F | F | H | - | -Cyc- |
| (54) | 7 | F | F | H | - | -Cyc- |
| (55) | 2 | Cl | H | H | - | -Cyc- |
| (56) | 3 | Cl | H | H | - | -Cyc- |
| (57) | 4 | Cl | H | H | - | -Cyc- |
| (58) | 5 | Cl | H | H | - | -Cyc- |
| (59) | 2 | Cl | F | H | - | -Cyc- |
| (60) | 3 | Cl | F | H | - | -Cyc- |
| (61) | 4 | Cl | F | H | - | -Cyc- |
| (62) | 5 | Cl | F | H | - | -Cyc- |
| (63) | 2 | $CF_3$ | H | H | - | -Cyc- |
| (64) | 3 | $CF_3$ | H | H | - | -Cyc- |
| (65) | 4 | $CF_3$ | H | H | - | -Cyc- |
| (66) | 5 | $CF_3$ | H | H | - | -Cyc- |
| (67) | 2 | $OCF_3$ | H | H | - | -Cyc- |
| (68) | 3 | $OCF_3$ | H | H | - | -Cyc- |
| (69) | 4 | $OCF_3$ | H | H | - | -Cyc- |
| (70) | 5 | $OCF_3$ | H | H | - | -Cyc- |

| | n | X | Y | Z | Q | A |
|---|---|---|---|---|---|---|
| (71) | 2 | $OCHF_2$ | H | H | - | -Cyc- |
| (72) | 3 | $OCHF_2$ | H | H | - | -Cyc- |
| (73) | 4 | $OCHF_2$ | H | H | - | -Cyc- |
| (74) | 5 | $OCHF_2$ | H | H | - | -Cyc- |

Es folgt ein Beispiel für ein Medium mit einem Gehalt an mindestens einer Verbindung der Formel I:

Beispiel A

Ein Gemisch aus
7 % p-(trans-4-Propylcyclohexyl)-benzonitril,
5 % p-(trans-4-Butylcyclohexyl)-benzonitril,
24 % p-(trans-4-Pentylcyclohexyl)-fluorbenzol,
14 % p-(trans-4-Heptylcyclohexyl)-fluorbenzol,
15 % 2-[trans-4-(3,4-Difluorphenyl)-cyclohexyl]-5-butyl-1,3-dioxan,
18 % 2-[trans-4-(p-fluorphenyl)-cyclohexyl]-5-butyl-1,3-dioxane und
17 % 2-[trans-4-(p-fluorphenyl)-cyclohexyl]-5-butyl-1,3-dioxan
zeigt einen hohen elektrischen Widerstand.

**Patentansprüche**

**1.** 2,5-Disubstituierte Heterocyclen der Formel I

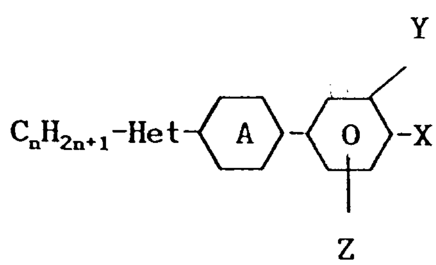

I

worin

n 1 bis 10,

Het

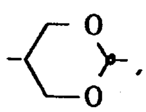 , 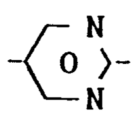 oder 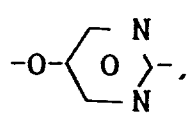 ,

Ring A trans-1,4-Cyclohexylen oder im Falle

Het = 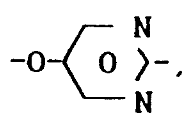 ,

X = $-OCF_3$ oder $-OCHF_2$ und/oder Y = Z = F auch 1,4-Phenylen,

X F, Cl, $-CF_3$, $-OCF_3$ oder $-OCHF_2$ und

Y und Z jeweils unabhängig voneinander H oder F

bedeuten, wobei im Falle X = F Y = F bedeutet.

2. Heterocyclen gekennzeichnet durch die Formel Ia

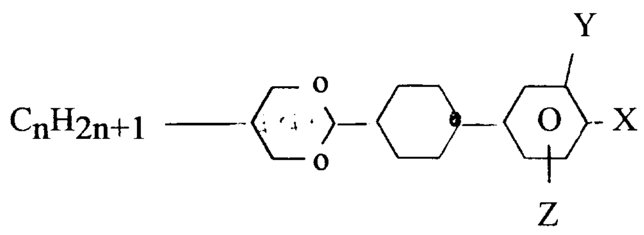

Ia

worin
n 1 bis 10, X F, Cl, $-CF_3$, $-OCF_3$ oder $-OCHF_2$ und Y und Z jeweils unabhängig voneinander H oder F bedeuten, wobei im Falle X = F Y = F bedeutet.

3. Heterocyclen gekennzeichnet durch die Formel Ib

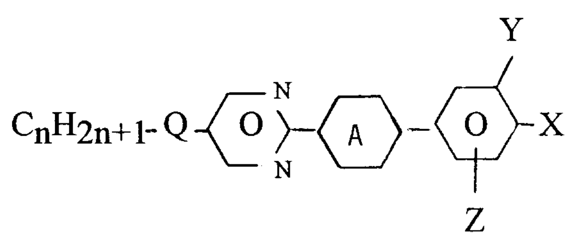

Ib

worin
n 1 bis 10, Q -O- oder eine Einfachbindung, A trans-1,4-Cyclohexylen oder 1,4-Phenylen, X F, Cl, $-CF_3$, $-OCF_3$ oder $-OCHF_2$ und Y und Z jeweils unabhängig voneinander H oder F bedeuten, wobei im Falle X = F Y = F bedeutet.

4. Heterocyclen nach Anspruch 1, 2 oder 3 dadurch gekennzeichnet, daß Y = F und Z = H oder F.

5. Verwendung der Heterocyclen der Formel I nach Anspruch 1 als Komponenten flüssigkristalliner Medien für elektrooptische Anzeigen.

6. Flüssigkristallines Medium für elektrooptische Anzeigen mit mindestens zwei flüssigkristallinen Komponenten, dadurch gekennzeichnet, daß mindestens eine Komponente ein Heterocyclus der Formel I nach Anspruch 1 ist.

7. Elektrooptische Anzeige auf der Basis einer Flüssigkristallzelle, dadurch gekennzeichnet, daß die Flüssigkristallzelle ein Medium nach Anspruch 6 enthält.

## Claims

1. 2,5-Disubstituted heterocyclic compounds of the formula I

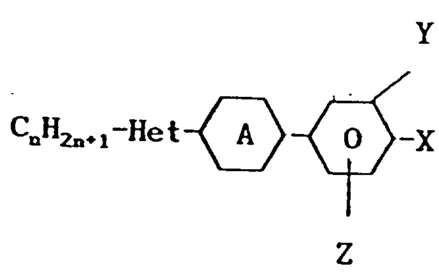

I

in which

n is 1 to 10,

Het is

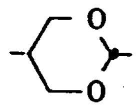, 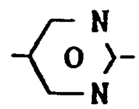 or 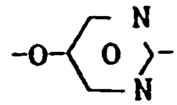 ,

ring A is trans-1,4-cyclohexylene or, in the case where

Het = 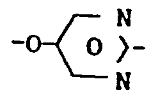 ,

X = $-OCF_3$ or $-OCHF_2$ and/or Y = Z = F, is alternatively 1,4-phenylene,

X is F, Cl, $-CF_3$, $-OCF_3$ or $-OCHF_2$, and

Y and Z are each, independently of one another, H or F

where, in the case where X = F, Y = F.

**2.** Heterocyclic compounds, characterized by the formula Ia

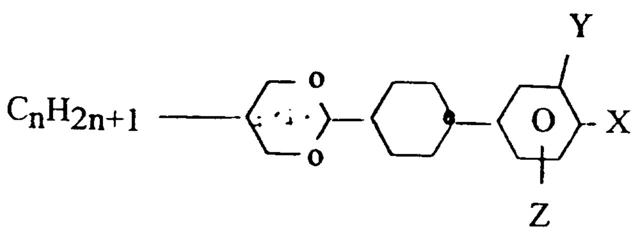

Ia

in which n is 1 to 10, X is F, Cl, $-CF_3$ or $-OCF_3$ or $-OCHF_2$ and Y and Z are each, independently of one another, H or F, where, in the case where X = F, Y = F.

**3.** Heterocyclic compounds, characrerized by the formula Ib

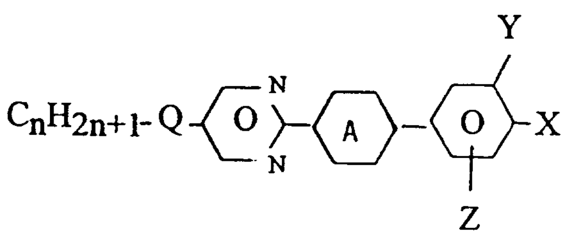

Ib

in which

n is 1 to 10, Q is -O- or a single bond, A is trans-1,4-cyclohexylene or 1,4-phenylene, X is F, Cl, $-CF_3$, $-OCF_3$ or $-OCHF_2$, and Y and Z are each, independently of one another, H or F, where, in the cas where X + F, Y = F.

**4.** Heterocyclic compounds according to Claim 1, 2 or 3, characterized in that Y = F and Z = H or F.

**5.** Use of the heterocyclic compounds of the formula I according to Claim 1 as components of liquid-crystalline media for electrooptical displays.

6. Liquid-crystalline medium for electrooptical displays containing at least two liquid-crystalline components, characterized in that at least one component is a heterocyclic compound of the formula I according to Claim 1.

7. Electrooptical display based on a liquid-crystal cell, characterized in that the liquid-crystal cell contains a medium according to Claim 6.

**Revendications**

1. Hétérocycles 2,5-disubstitués de formule I

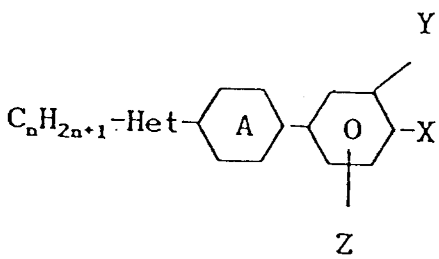

I

dans laquelle
n représente 1 à 10,
Het représente

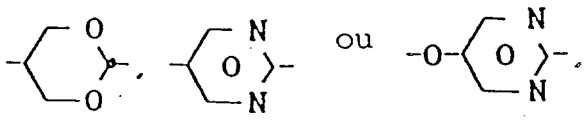

Cycle A représente un trans-1,4-cyclohexylène ou,
dans le cas où

Het = 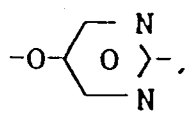

X = $-OCF_3$ ou $-OCHF_2$
et/ou Y = Z = F, un 1,4-phénylène aussi,
X représente F, Cl, $-CF_3$, $-OCF_3$ ou $-OCHF_2$ et
Y et Z représentent chacun indépendamment H ou F, et quand X = F on a Y = F.

2. Hétérocycles caractérisés par la formule Ia

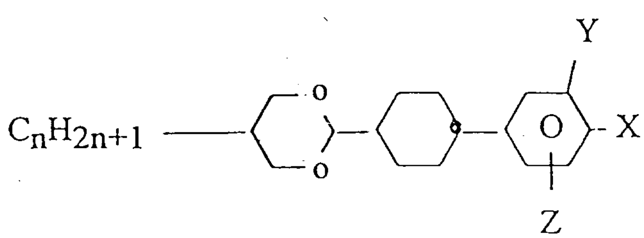

Ia

dans laquelle

n représente 1 à 10, X représente F, Cl, $-CF_3$, $-OCF_3$ ou $-OCHF_2$ et Y et Z représentent chacun indépendamment H ou F, et quand X = F on a Y = F.

**3.** Hétérocycles caractérisés par 1a formule Ib

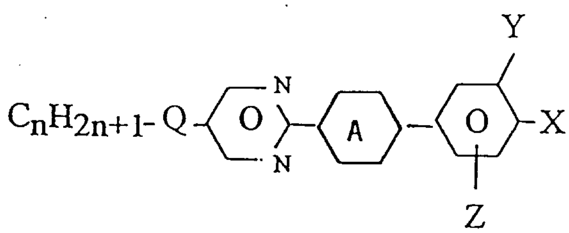

Ib

dans laquelle

n représente 1 à 10, Q représente -O- ou une liaison simple, A un trans-1,4-cyclohexylène ou 1,4-phénylène, X représente F, Cl, $-CF_3$, $-OCF_3$ ou $-OCHF_2$ et Y et Z représentent chacun indépendamment H ou F, et quand X = F on a Y = F.

**4.** Hétérocycles selon la revendication 1, 2 ou 3, caractérisés en ce que Y = F et Z H ou F.

**5.** Utilisation des hétérocycles de formule I, selon la revendication 1, comme composants de milieux à cristaux liquides pour des affichages électrooptiques.

**6.** Milieu de cristaux liquides pour affichages électrooptiques avec au moins deux composants de cristaux liquides, caractérisé en ce qu'au moins un composant est un hétérocycle de formule I selon 1a revendication 1.

**7.** Affichage électrooptique à base d'une cellule de cristaux liquides, caractérisé en ce que la cellule de cristaux liquides comprend un milieu selon la revendication 6.